# EUROPÄISCHE PATENTSCHRIFT

(11) **EP 1 496 859 B1**
(45) Veröffentlichungstag und Bekanntmachung des Hinweises auf die Patenterteilung: **05.11.2008**
(21) Anmeldenummer: 03712116.7
(22) Anmeldetag: 31.03.2003
(51) Int. Cl.: A61K 9/08, A61K 9/00, A61K 47/20

(54) **WÄSSRIGE FORMULIERUNGEN VON (2-HYDROXYMETHYL-INDANYL-4-OXY)-PHENYL-4,4,4- TRIFLUORBUTAN-1-SULFONAT**
AQUEOUS FORMULATIONS OF (2-HYDROXYMETHYL-INDANYL-4-OXY)-PHENYL-4,4,4-TRIFLUOROBUTANE-1-SULFONATE
COMPOSITIONS AQUEUSES DE (2-HYDROXYMETHYLINDANYL-4-OXY)-PHENYL-4,4,4-TRIFLUORBUTANE-1-SULFONATE

(30) Priorität: 11.04.2002 DE 10215942
(43) Veröffentlichungstag der Anmeldung: 19.01.2005
(73) Patentinhaber: Bayer HealthCare AG, 51368 Leverkusen (DE)
(72) Erfinder: KÜHN, Bernd, 60323 Frankfurt (DE); BRÜCK, Antje, 78464 Konstanz (DE); KATAKAWA, Yoshifumi, Kusatsu-shi, Shiga 525-0025 (JP); YASUI, Masami, Koka-gun, Shiga 528-0004 (JP)
(86) Internationale Anmeldenummer: PCT/EP2003/003327
(87) Internationale Veröffentlichungsnummer: WO 2003/084506

(56) Entgegenhaltungen:
- WO-A-98/37061
- MAULER FRANK ET AL: "Characterization of the diarylether sulfonylester (-)-(R)-3-(2-hydroxymethylindanyl-4-oxy)ph enyl-4,4,4-trifluoro-1-sulf onate (BAY 38-7271) as a potent cannabinoid receptor agonist with neuroprotective properties." THE JOURNAL OF PHARMACOLOGY AND EXPERIMENTAL THERAPEUTICS. UNITED STATES JUL 2002, Bd. 302, Nr. 1, Juli 2002 (2002-07), Seiten 359-368, XP002246549 ISSN: 0022-3565
- PEKKA JARHO ET AL: "Hydroxypropyl-beta-cyclodextrin and its combination with hydroxypropyl-methylcellulose increases aqueous solubility of A9-tetrahydrocannabinol" LIFE SCIENCES, PERGAMON PRESS, OXFORD, GB, Bd. 63, Nr. 26, 1998, Seiten 381-384, XP002963822 ISSN: 0024-3205
- THORSTEINN LOFTSSON ET AL: "Pharmaceutical Applications of Cyclodextrins. 1. Drug Solubilization and Stabilization" JOURNAL OF PHARMACEUTICAL SCIENCES, AMERICAN PHARMACEUTICAL ASSOCIATION. WASHINGTON, US, Bd. 85, Nr. 10, 1. Oktober 1996 (1996-10-01), Seiten 1017-1025, XP002080430 ISSN: 0022-3549

## Beschreibung

Die vorliegende Erfindung betrifft (-)-(R)-3-(2-Hydroxymethyl-indanyl-4-oxy)-phenyl-4,4,4-trifluorbutan-l-sulfonat enthaltende wässrige Formulierungen, die sich als Infusionslösungen oder als Konzentrat zur Herstellung solcher Infusionslösungen eignen

(-)-(R)-3-(2-Hydroxymethylindanyl-4-oxy)-phenyl-4,4,4-trifluorbutan-1-sulfonat ist eine Verbindung der Formel

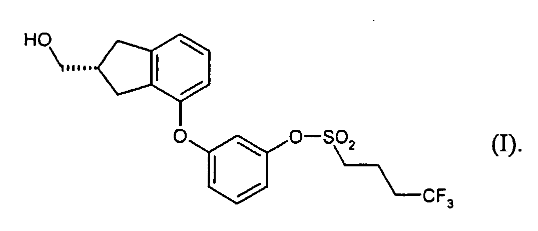

Als Cannabinoid-Rezeptor-Agonist eignet sich die Verbindung (I) zur Prävention und Behandlung von Schlaganfall und Schädel-Hirn-Trauma; sie wurde erstmals in Beispiel 278 der WO 98/37061 beschrieben. Für parenterale Applikation geeignete pharmazeutische wässrige Zubereitungen werden in der WO 98/37061 allerdings nicht offenbart. Da bei der akuten Behandlung von Schlaganfall und Schädel-Hirn-Trauma das Arzneimittel vorteilhafterweise als Infusionslösung verabreicht wird, bestand ein Bedarf an wässrigen Formulierungen, die Verbindung (I) enthalten und diesem Einsatzzweck gerecht werden.

Wässrige Formulierungen der Verbindung (I) zeigen merkwürdigerweise eine inhomogene Konzentrationsverteilung. Dadurch ist insbesondere bei niedrigen Wirkstoffkonzentrationen von wenigen Milligramm pro Liter davon auszugehen, dass eine auf die Dosis bezogene, konstante Infusionsrate über die gesamte Infusionsdauer nicht gewährleistet werden kann. Die damit verbundenen Nachteile liegen auf der Hand.

Für einzeldosierte Arzneiformen, u.a. parenterale Pulver und Suspensionen zur Injektion, fordern die Arzneibücher (Ph. Eur. 4, 2002) die Prüfung auf Gehaltseinheitlichkeit, eine möglichst geringe Abweichung von maximal ± 15 % vom Durchschnitt des Wirkstoffgehalts.

Überraschenderweise wurde gefunden, dass der Zusatz von Cyclodextrin zu wässrigen Formulierungen zu gleichmäßiger Konzentration führte.

Gegenstand der Erfindung sind somit wässrige Formulierungen enthaltend Verbindung (I) und Cyclodextrin.

Cyclodextrine und Verfahren zu ihrer Herstellung sind aus US 3,453,259, US 3,459,731, WO 97/39770, US 5,670,530, WO 96/32135, EP-B 149 197 und US 4,727,064 bekannt. Cyclodextrine sind cyclische Oligosaccharide, die beim Abbau von Stärke durch Cyclodextrin-Glykosyltransferasen gebildet werden.

β-Cyclodextrine enthalten sieben α-1,4-verknüpfte Glucose-Einheiten. Die in diesem Molekül enthaltenden 21 Hydroxygruppen können ganz oder teilweise z.B. mit gegebenenfalls substituierten aliphatischen C₂-C₆-Gruppen, vorzugsweise mit Hydroxypropyl- oder Sulfobutylgruppen, substituiert werden. Die verwendeten Cyclodextrine weisen dabei vorzugsweise einen durchschnittlichen Substitutionsgrad (DS) pro Molekül von 1 bis 10, insbesondere von 3 bis 8 auf.

Der Begriff "Cyclodextrin" im Sinne der Erfindung umfasst die unsubstituierten, die teilweise und die vollständig substituierten Cyclodextrine, insbesondere Hydroxypropyl- und Sulfobutyl-substituierte β-Cyclodextrine.

Überraschenderweise zeigt sich außerdem, dass physiologisch verträgliche Säuren die Lagerstabilität der wässrigen Formulierungen erhöhen können.

Beispiele solcher physiologisch verträglichen Säuren umfassen Mineralsäuren wie z.B. Salzsäure, Schwefelsäure, ein- bis 4-basische gesättigte und ungesättigte C₂-C₁₀-Carbonsäuren wie z.B. Essigsäure, Bernsteinsäure, Maleinsäure, Fumarsäure, C₂-C₆-Hydroxycarbonsäuren wie z.B. Äpfelsäure, Citronensäure Glycolsäure, Milchsäure, Weinsäure, Zimtsäure, C₃-C₆-Ketocarbonsäuren wie z.B. Brenztraubensäure, ein- oder zweibasische C₂-C₁₀-Aminosäuren wie z.B. Alanin, Asparaginsäure, Glutaminsäure, Glycin, Isoleucin, Leucin, Lysin, Methionin, Phenylalanin, Prolin, Serin, Threonin, Valin, C₆-C₁₂-Amidocarbonsäuren wie z.B. Hippursäure, C₄-C₁₀-Lactone wie z.B. Ascorbinsäure, und deren Mischungen. Bevorzugt sind Milch- und Citronensäure; besonders bevorzugt ist Citronensäure.

Ein bevorzugter pH-Bereich für die erfindungsgemäßen wässrigen Formulierungen beträgt 2 bis 6, insbesondere 3 bis 5, und speziell etwa 3,5 bis 4,5.

Zur Herstellung einer isotonen Lösung können die erfindungsgemäßen Formulierungen für diesen Zweck geeignete Verbindungen wie beispielsweise Glucose, Mannit, vorzugsweise Natriumchlorid enthalten. Als isoton wird eine Lösung bezeichnet, wenn sie einen osmotischen Druck von 250 bis 500, vorzugsweise 270 bis 350 mOsmol/kg aufweist.

Bevorzugte erfindungsgemäße isotone Formulierungen enthalten 5 bis 15, bevorzugt 7 bis 13 und besonders bevorzugt 8 bis 10 g/L Natriumchlorid, bezogen auf gebrauchsfertige Formulierung.

Den erfindungsgemäßen Formulierungen kann man weiterhin physiologisch verträgliche organische Lösungsmittel, beispielsweise Polyethylenglycole, Propylenglycol, Glycofurol, Glycerol oder - bevorzugt - Alkohole, insbesondere Ethanol zusetzen.

Die erfindungsgemäßen Formulierungen können im allgemeinen 0,05 bis 2 vorzugsweise 0,1 bis 1,5 und insbesondere etwa 0,6 bis 1,0 g/L organisches Lösungsmittel, bezogen auf gebrauchsfertige Formulierung, enthalten.

Die erfindungsgemäßen Formulierungen können als **gebrauchsfertige** Infusionslösungen oder als wässrige Konzentrate vorliegen, aus denen dann die Infusionslösungen durch Zugabe von Wasser oder isotonischer Elektrolytlösung hergestellt werden können. Diese erfindungsgemäßen Konzentrate können die Verbindung (I) in einer Konzentration von 0,002 bis 9,0, vorzugsweise von 0,01 bis 0,05, besonders bevorzugt von 0,025 g/L enthalten. Die Konzentrate können Cyclodextrin in Konzentrationen von 4 bis 550, vorzugsweise von 20 bis 200, besonders bevorzugt von 50 g/L enthalten. Aus den Konzentraten kann leicht und schnell unter sterilen Bedingungen eine homogene Lösung hergestellt werden, die sich direkt zur Anwendung, beispielsweise als Infusionslösung, eignet.

Die erfindungsgemäße Formulierung kann Cyclodextrin in 0,1 bis 60, bevorzugt 1 bis 30, besonders bevorzugt 1 bis 10, insbesondere 2 g/L bezogen auf die gebrauchsfertige Formulierung enthalten.

Die Löslichkeit der Verbindung (I) in Wasser beträgt bei 25°C 0,002 g/L.

Die erfindungsgemäße zur Infusion **gebrauchsfertige** Formulierung kann eine Wirkstoffkonzentration von 0,00005 bis 0,002, vorzugsweise 0,0001 bis 0,002, insbesondere 0,0005 bis 0,0015 und ganz speziell etwa 0,001 g Verbindung (I)/L Lösung enthalten.

Die erfindungsgemäßen Formulierungen können einfach durch Mischen und Lösen der Komponenten hergestellt werden.

Im allgemeinen hat es sich als vorteilhaft erwiesen, die Verbindung (I) in Gesamtmengen von etwa 0,001 bis etwa 240, bevorzugt 0,01 bis 24 µg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von Art und Körpergewicht des behandelten Patienten, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Weiterer Gegenstand der Erfindung ist ein Applikations-Set bestehend aus einem Behälter enthaltend die wässrige Formulierung und einem Infusionsgerät. Das Infusionsgerät besteht im einfachsten Fall aus einer Kanüle, Verbindungsschläuchen, und einer Tropfkammer. An den Verbindungsschläuchen können eine Infusionspumpe sowie Regulationshähne angeschlossen sein. Weiterhin kann die Applikation mittels Spritzenpumpen aus Infusionsspritzen mit angeschlossenen Verbindungsschläuchen erfolgen.

Die produktberührenden Materialien des Applikations-Sets können beispielsweise aus Polyethylen (PE), Polypropylen (PP), Polyamiden, Polyestern oder deren Copolymerisate, Acrylnitril-Butadien-Styrol-Copolymeren, Polypropylen/Styrol-Ethylen-Butylen-Styrol, vorzugsweise aus Polyolefinen, besonders bevorzugt aus Polyethylen bestehen.

### Beispiele:

### 1) Beispiel einer gebrauchsfertigen Infusionsformulierung auf Basis von Hydroxypropyl-β-cyclodextrin

### Zusammensetzung (in g/L)

| | |
|---|---|
| Verbindung (I) | 0,001 |
| Hydroxypropyl-β-cyclodextrin (®Cavitron 82004, Cerestar) | 2 |
| Natriumchlorid | 9 |
| Ethanol f. Inj. | 0,8 |
| Citronensäure | 0,016 |
| Wasser | 993,383 |

Herstellung: Eine Lösung der Verbindung (I) in Ethanol wird unter Rühren zu einer wässrigen Lösung von Hydroxypropyl-β-cyclodextrin und Natriumchlorid gegeben. Der pH-Wert wird mit Citronensäure auf ca. 4 eingestellt. Die Lösung wird sterilfiltriert, in Glasflaschen zu 250 mL abgefüllt, mit Gummistopfen und Bördelkappen verschlossen und anschließend im Dampfautoklaven bei 121°C für 20 min sterilisiert.

### 2) Beispiel einer gebrauchsfertigen Infusionsformulierung auf Basis von Sulfobutylether-β-cyclodextrin

### Zusammensetzung (in g/L)

| | |
|---|---|
| Verbindung (I) | 0,001 |
| Sulfobutylether-β-cyclodextrin (®Captisol, CyDex) | 2 |
| Natriumchlorid | 9 |
| Ethanol f. Inj. | 0,8 |
| Citronensäure | 0,016 |
| Wasser | 993,383 |

Herstellung: Eine Lösung der Verbindung (I) in Ethanol wird unter Rühren zu einer wässrigen Lösung von Sulfobutylether-β-cyclodextrin und Natriumchlorid gegeben. Der pH-Wert wird mit Citronensäure auf ca. 4 eingestellt. Die Lösung wird sterilfiltriert, in Glasflaschen zu 250 mL abgefüllt, mit Gummistopfen und Bördelkappen verschlossen und anschließend im Dampfautoklaven bei 121°C für 20 min sterilisiert.

### 3) Beispiel eines Konzentrats zur Herstellung einer Infusionsformulierung

### Zusammensetzung (in g/L)

| | |
|---|---|
| Verbindung (I) | 0,025 |
| Hydroxypropyl-β-cyclodextrin (®Cavitron 82004, Cerestar) | 50 |
| Natriumchlorid | 9 |
| Ethanol f. Inj. | 0,8 |
| Citronensäure | 0,016 |
| Wasser | ad 1,0L |

Herstellung: Eine Lösung der Verbindung (I) in Ethanol wird unter Rühren zu einer wässrigen Lösung von Hydroxypropyl-β-cyclodextrin und Natriumchlorid gegeben. Der pH-Wert wird mit Citronensäure auf ca. 4 eingestellt. Die Lösung wird sterilfiltriert, in Glasflaschen zu 10 mL abgefüllt, mit Gummistopfen und Bördelkappen verschlossen und anschließend im Dampfautoklaven bei 121 °C für 20 min sterilisiert.

Vor Anwendung werden 10 ml Konzentrat mit 240 ml physiologischer Kochsalzlösung gemischt. Es ergibt sich eine gebrauchsfertige Infusionslösung der Wirkstoffkonzentration 0,001 g/L.

## Patentansprüche

1. Wässrige Formulierungen enthaltend (-)-(R)-3-(2-Hydroxymethyl-indanyl-4-oxy)-phenyl-4,4,4-trifluorbutan-1-sulfonat (I) und Cyclodextrin.

2. Formulierungen nach Anspruch 1, enthaltend 0,00005 bis 9,0 g/L der Verbindung (I) und 0,1 bis 550 g/L Cyclodextrin.

3. Formulierungen nach einem der vorhergehenden Ansprüche, enthaltend 0,0001 bis 0.050 g/L der Verbindung (I) und 0,2 bis 200 g/L Cyclodextrin.

4. Formulierungen nach einem der vorhergehenden Ansprüche, enthaltend 0,0005 bis 0,025 g/L der Verbindung (I) und 1 bis 50 g/L Cyclodextrin.

5. Formulierungen nach einem der vorhergehenden Ansprüche, die einen pH-Wert von 2 bis 6 aufweisen.

6. Formulierungen nach einem der vorhergehenden Ansprüche, enthaltend mindestens eine physiologisch verträgliche Säure.

7. Formulierungen nach Anspruch 6, die als physiologisch verträgliche Säure Citronensäure enthalten.

8. Formulierungen nach einem der vorhergehenden Ansprüche, enthaltend 8 bis 10 g/L Natriumchlorid, bezogen auf gebrauchsfertige Formulierung.

9. Formulierungen nach einem der vorhergehenden Ansprüche, enthaltend 0,05 bis 2 g/L Ethanol, bezogen auf gebrauchsfertige Formulierung.

10. Applikations-Set bestehend aus
a) einem Behälter enthaltend die wässrige Formulierung nach den Ansprüchen 1 bis 9,
b) Infusionsgerät, wobei zumindest die produktberührenden Teile aus Polyethylen, Polypropylen, Polyester, Polyamid, Acrylnitril-Butadien-Styrol-Copolymeren, Polypropylen/Styrol-Ethylen-Butylen-Styrol oder deren Copolymerisaten bestehen.

## Claims

1. Aqueous formulations comprising (-)-(R)-3-(2-hydroxymethylindanyl-4-oxy)phenyl 4,4,4-trifluorobutane-1-sulfonate (I) and cyclodextrin.

2. Formulations according to claim 1, comprising from 0.00005 to 9.0 g/l of the compound (I) and from 0.1 to 550 g/l cyclodextrin.

3. Formulations according to either of the preceding claims, comprising from 0.0001 to 0.050 g/l of the compound (I) and from 0.2 to 200 g/l cyclodextrin.

4. Formulations according to any of the preceding claims, comprising from 0.0005 to 0.025 g/l of the compound (I) and from 1 to 50 g/l cyclodextrin.

5. Formulations according to any of the preceding claims, which have a pH of from 2 to 6.

6. Formulations according to any of the preceding claims, comprising at least one physiologically tolerated acid.

7. Formulations according to claim 6, which comprise citric acid as physiologically tolerated acid.

8. Formulations according to any of the preceding claims, comprising from 8 to 10 g/l sodium chloride based on the formulation ready for use.

9. Formulations according to any of the preceding claims, comprising from 0.05 to 2 g/l ethanol based on the formulation ready for use.

10. Administration kit consisting of
a) a container comprising the aqueous formulation according to claims 1 to 9,
b) infusion apparatus, where at least the parts which come into contact with the product consist of polyethylene, polypropylene, polyester, polyamide, acrylonitrile-butadiene-styrene copolymers, polypropylene/styrene-ethylene-butylene-styrene or copolymers thereof.

## Revendications

1. Formulations aqueuses contenant du (-)-(R)-3-(2-hydroxyméthyl-indanyl-4-oxy)-phényl-4,4,4-trifluorobutane-1-sulfonate (I) et de la cyclodextrine.

2. Formulations suivant la revendication 1, contenant 0,00005 à 9,0 g/l de composé (I) et 0,1 à 550 g/l de cyclodextrine.

3. Formulations suivant l'une des revendications précédentes, contenant 0,0001 à 0,500 g/l de composé (I) et 0,2 à 200 g/l de cyclodextrine.

4. Formulations suivant l'une des revendications précédentes, contenant 0,0005 à 0,025 g/l de composé (I) et 1 à 50 g/l de cyclodextrine.

5. Formulations suivant l'une des revendications précédentes, qui présentent une valeur de pH de 2 à 6.

6. Formulations suivant l'une des revendications précédentes, contenant au moins un acide physiologiquement acceptable.

7. Formulations suivant la revendication 6, qui contiennent de l'acide citrique comme acide physiologiquement acceptable.

8. Formulations suivant l'une des revendications précédentes, contenant 8 à 10 g/l de chlorure de sodium, sur la base de la préparation prête à l'emploi.

9. Formulations suivant l'une des revendications précédentes, contenant 0,05 à 2 g/l d'éthanol, sur la base de la préparation prête à l'emploi.

10. Nécessaire d'application, constitué
a) d'un récipient contenant la formulation aqueuse suivant les revendications 1 à 9,
b) d'un appareil de perfusion, tout au moins les parties entrant en contact avec le produit étant constituées de polyéthylène, polypropylène, polyester, polyamide, copolymères acrylonitrile-butadiène-styrène, polypropylène/styrène-éthylène-butylène-styrène ou leurs produits de copolymérisation.
